# EUROPÄISCHE PATENTSCHRIFT

(11) **EP 1 319 010 B1**
(45) Veröffentlichungstag und Bekanntmachung des Hinweises auf die Patenterteilung: **17.12.2003**
(21) Anmeldenummer: 01957919.2
(22) Anmeldetag: 05.07.2001
(51) Int. Cl.: C07D 513/04, A61K 31/505, A61K 31/54, A61P 29/00, C07D 498/04, C07D 487/04

(54) **TRICYCLISCHE MERCAPTOMETHYLSUBSTITUIERTE 2,3-DIHYDRO-CHINAZOLIN-5-ONE UND 2,3-DIHYDRO-BENZO-[1,2,4]-THIADIAZIN-5,5-DIOXIDE ALS MATRIX METALLOPROTEINASE (MMP) INHIBITOREN**
TRICYCLIC MERCAPTOMETHYL-SUBSTITUTED 2,3-DIHYDRO-QUINAZOLIN-5-ONES AND 2,3-DIHYDRO-BENZO-[1,2,4]-THIADIAZIN-5,5-DIOXIDES AS MATRIX METALLOPROTEINASE (MMP) INHIBITORS
2,3-DIHYDRO-QUINAZOLIN-5-ONES ET 2,3-DIHYDRO-BENZO-[1,2,4]-THIADIAZIN-5,5-DIOXYDES SUBSTITUES PAR UN MERCAPTOMETHYLE TRICYCLIQUE ET UTILISES COMME INHIBITEURS DE LA MATRICE METALLOPROTEINASE (MMP)

(30) Priorität: 21.09.2000 DE 10046728
(43) Veröffentlichungstag der Anmeldung: 18.06.2003
(73) Patentinhaber: IBFB GmbH Privates Institut für biomedizinische Forschung und Beratung, 04229 Leipzig (DE)
(72) Erfinder: KLAUSMEIER, Uwe, 33330 Güntersloh (DE); HEINICKE, Jochen, 04229 Leipzig (DE); ARKONA, Christoph, 04229 Leipzig (DE)
(74) Vertreter: Schüssler, Andrea, Dr.
(86) Internationale Anmeldenummer: EP0107710
(87) Internationale Veröffentlichungsnummer: WO02024710

(56) Entgegenhaltungen:
- DD-A- 298 784
- DE-A- 2 252 122
- US-A- 5 326 760

## Beschreibung

Die Erfindung betrifft Vertreter der Substanzklassen der Thiazolo[2,3-b]chinazolinone, Oxazolo[2,3-b]chinazolinone, Imidazo[2,1-b]chinazolinone sowie der Benzothiadiazin-5,5-dioxide der allgemeinen Formeln Ia und Ib, 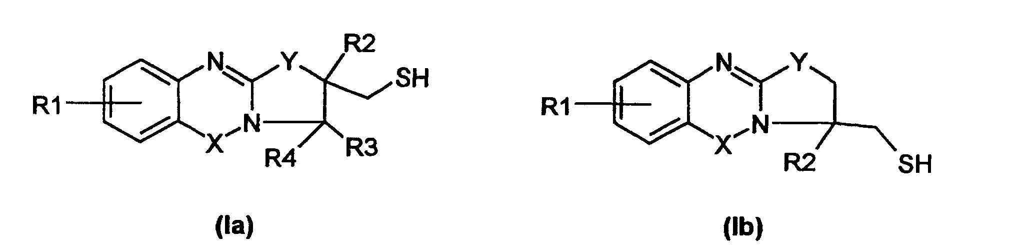 worin bedeuten:
R1 = Wasserstoff, Methyl, Methoxy, Dimethoxy, Hal (Hal = Fluor, Chlor, Brom), Carboxy
R2, R3, R4 = Wasserstoff, Methyl
X = Carbonyl, Sulfonyl
Y = S, O, NH
sowie deren Tautomere und Salze und ihren Einsatz als Matrix Metalloproteinase (MMP)- Inhibitoren.

Es ist das Ziel der Erfindung, Arzneimittel zur Behandlung zahlreicher Erkrankungen, wie z.B. Krebs, Rheuma, unspezifische Entzündungsreaktionen u.a. beim Sonnenbrand und allergischen Reaktionen zu entwickeln

Chinazoline, Chinazolinone und Chinazolindione sind Gegenstand einer intensiven pharmazeutischen Forschung. Ihre Eignung als Wirkstoffe und Synthesebausteine ist unbestritten.

Nur wenig ist demgegenüber zu Synthesen und Wirkungen von mercaptoalkylsubstituierten Chinazolinonen und deren bioisosteren Analoga aus der Gruppe der Benzothiadiazin-1,1-dioxide bekannt. Die Wirksamkeit schwefelsubstituierter Chinazolindione, speziell von 3-(Mercaptoalkyl17.09.00)chinazolin2,4(1H,3H)-dionen als Pharmazeutika ist von Leistner u.a. erkannt worden (DD 298 784). Es wurde festgestellt, dass eine immunstimulatorische und antivirale Wirksamkeit von Vertretern dieser Substanzgruppe gegeben ist.

Verbindungen mit einem tricyclischen Grundkörper entsprechend der Formeln la und Ib, allerdings anderem Substitutionsmuster wurden vereinzelt beschrieben und zeigten spezielle pharmakologische Wirkungen *(Chern, J.-W et al.: Studies on Quinazolines and 1,2,4-Benzothiadiazine 1,1-Dioxides. 8. Synthesis and pharmacological evaluation of tricyclic fused Quinazolines and 1,2,4-Benzothiadiazine 1,1-dioxides as potential α1-Adrenoceptor Antagonists, J. Med. Chem. 41 (1998) 3128-3141; Liu, K.-C. u. Hsu, L-Y.: Synthese und antihypertensive Aktivität einiger Chinazolinon-Derivate, Arch. Pharm. 318 (1985) 502- 505)* . Mercaptoalkyisubstituierte Tricyclen entsprechend der allgemeinen Formeln Ia und Ib hingegen sind in der Fachliteratur bislang nicht beschrieben.

Auf dem Gebiet der Entwicklung effizienter, niedermolekularer, und nichtproteinogener MMP Inhibitoren wird weltweit intensiv geforscht. Es ist bekannt, dass physiologisch die enzymatische Aktivität der MMPs unter einer strikten, abgestimmten Regulation zwischen Aktivierung und Hemmung steht. Hierzu existieren im Organismus spezielle Proteine, die sog. Tissue Inhibitors of Matrixmetalloproteinases (TIMP's), die in der Lage sind, die Aktivität von MMPs schnell und effizient zu hemmen *(Nagase, H. et al.: Engineering of selective TIMPs. 1-11; In: Inhibition of Matrix Metalloproteinases - Therapeutic Application (Eds. Greenwal,d RA., Zucker, S., Golub, LM.) Ann NY Acad Sci 878 (1999)*. Eine ungebremste enzymatische Aktivität dieser Enzyme führt besonders bei den rheumatischen Erkrankungen zum Abbau der Knorpelsubstanz und - pathologisch bedeutsam - zu chronisch-schmerzhaften Veränderungen an den Gelenken *(Goldbach-Mansky, R. et al.: Active synovial matrix metalloproteinase-2 is associated with radiographic erosions in patients with early synovitis. Arthritis Res 2 (2000) 145-153).*

Ein weiteres Beispiel für die pathologische Wirkung von MMPs ist bei der Invasion und Metastasierung von Tumoren gegeben. Freigesetzte und aktivierte MMPs schneiden einen Weg durch das dichtes kollagenes Bindegewebe und insbesondere auch durch die Basalmembran der Gefäße und ermöglichen es dadurch den Krebszellen aus dem Tumorverband auszuwandern, in das Gefäßsystem einzuwandern und an anderer Stelle Tochtergeschwülste zu bilden. MMPs spielen eine weitere entscheidende Rolle bei der Blutgefäßversorgung des wachsenden Tumors, indem sie den Weg für die neugebildeten Blutgefäße durch das kollagene Bindegewebe freischneiden und somit für diese Vaskularisation des wachsenden Tumors verantwortlich sind *(Shapiro, SD.: Matrix metalloproteinase degradation of extracellular matrix: biological consequences. Current Opinion in Cell Biology 10 (1998) 602-608).*

Als weiteres relevantes medizinisches Ergebnis unzureichend gebremster Wirkung von MMPs ist das UV-induzierte Erythem zu nennen, das u.a. als Folge einer intensiven Sonnenbestrahlung auftritt. Durch die energiereichen UV-Strahlen des Sonnenlichtes bzw. von Bräunungsgeräten werden u.a. die inaktiven Prokollagenasen in der bestrahlten Haut aktiviert, die in der Folge Kollagen des Bindegewebes und der Blutkapillaren spalten und dadurch für die Symptome eines Sonnenbrandes verantwortlich zeichnen.

Bei diesen exemplarisch aufgezeigten pathologischen Wirkungen der ungebremsten enzymatischen Aktion von MMPs, können deren Folgeerscheinungen durch stabile MMP- Inhibitoren verhindert bzw. wesentlich gemildert werden. Es ist faszinierend, die Vorstellung zu realisieren, diese Enzyme durch spezifische Inhibitoren gezielt zu hemmen, um dadurch zum Beispiel eine fortschreitende Knorpeldestruktion bei Erkrankungen des rheumatischen Formenkreises zu unterbrechen, oder das Wachstum bzw. die Metastasierung von Tumoren zu verhindern.

Es sind bereits zahlreiche Verfahren zur Gewinnung von Verbindungen mit MMP-inhibierender Wirkung bekannt. Diese Wirkstoffe der ersten Generation besitzen in aller Regel eine proteinogene Struktur und weisen eine strukturelle Verwandtschaft zu natürlichen Inhibitoren auf, bei denen es sich um spezielle Proteine handelt. Diese proteinogenen oder pseudoproteinogenen Substratanaloga besitzen als Strukturelement eine zinkbindende Gruppe, die das Zinkion im aktiven Zentrum der MMPs chelatisiert.

Bezüglich einer therapeutischen Anwendung weisen alle derartigen proteinogenen bzw. pseudoproteinogenen Wirkstoffe eine Reihe von Nachteilen auf, wie ungenügende Resorbierbarkeit, in aller Regel kurze Halbwertszeiten, eine nur geringe Stabilität, sowie häufig unerwünschte Nebenwirkungen *(Inhibition of Matrix Metalloproteinases - Therapeutic Application (Eds. Greenwald, RA., Zucker, S., Golub, LM.) Ann NY Acad Sci 878 (1999))*.

Weitere Entwicklungen auf diesem Gebiet erbrachten beispielsweise Phosphonamid-Inhibitoren, Piperazin-Inhibitoren, Sulfonamid-Inhibitoren, Carbamat-Inhibitoren, Diazepin-Inhibitoren, Tetracyclin-Inhibitoren und nicht zuletzt Hydroxamat-Inhibitoren *(Skotnick,i JS. et al.: Design and synthetic considerations of matrix metalloproteinase inhibitors. 61-72 In: Inhibition of Matrix Metalloproteinases - Therapeutic Application (Eds. Greenwald, RA., Zucker, S., Golub, LM.) Ann NY Acad Sci 878 (1999))*. Die meisten dieser entwickelten Inhibitoren weisen zwar *in vitro* beeindruckende Hemmwirkungen und Spezifitäten auf, zeigten aber bei *in vivo* Anwendungen im Tierversuch und beim Menschen eine Reihe von schwerwiegenden Nachteilen. Im Vordergrund standen hierbei zytotoxische Reaktionen auf eine Vielzahl von Zellen, eine schlechte Bioverfügbarkeit und unerwünschte Nebenwirkungen, insbesondere eine negative Beeinflussung des Bewegungsapparates.

Es besteht daher ein Bedarf an Arzneimitteln mit nichtproteinogener Struktur, welche die Nachteile der Wirkstoffe, die bisher am Markt sind, nicht aufweisen. Insbesondere besteht Bedarf an neuen Wirkstoffen mit MMP-inhibierender Wirkung, die ausreichend stabil und gut resorbierbar sind, bessere pharmakokinetische Eigenschaften besitzen und vor allen Dingen keine unerwünschten Nebenwirkungen und zytotoxische Reaktionen aufweisen.
Die Erfindung hat deshalb die Aufgabe, neue chemische Substanzen nichtproteinogener Struktur aufzufinden, die eine MMP-inhibierende Wirkung zeigen. Es ist weiter Aufgabe der Erfindung, Verfahren zur Herstellung solcher Verbindungen als auch entsprechende Arzneimittel zur Verfügung zu stellen, welche diese Verbindungen enthalten.

Die Aufgabe wird anspruchsgemäß gelöst.

Die erfindungsgemäßen, erstmals hergestellten Verbindungen der allgemeinen Formeln Ia und Ib aus der Klasse der tricyclischen mercaptoalkylsubstituierten 2,3-Dihydro-chinazolin-5-one und deren bioisosterer 2,3-Dihydro-benzo-[1,2,4]-thiadiazin-5,5-dioxide zeigen überraschende, deutliche und damit pharmakologisch interessante MMP-inhibierende Wirkungen, welche aus bisher bekannten Struktur-Wirkungsbeziehungen nicht ableitbar sind.

Durch Testung dieser Inhibitoren entsprechend der allgemeinen Formeln Ia und Ib an unterschiedlichen humanen MMPs (MMP-2, rekombinante katalytische Domäne der MMP-8, MMP-9, rekombinante katalytische Domäne der MMP-14) werden Spezifitäten der erfindungsgemäßen Verbindungen belegt. Spezifische Inhibitoren würden besonders beim Einsatz als Arzneimittel nur die gewünschten Zielenzyme beeinflussen und nicht das ausbalancierte Zusammenspiel der anderen MMPs stören und somit mögliche unerwünschte Nebenwirkungen vermeiden helfen.

Die erfindungsgemäßen Mercaptane der allgemeinen Formeln Ia und Ib werden nach literaturbekannten Verfahren durch Umsetzung der analogen Jodide, Bromide, Chloride oder Tosylate der allgemeinen Formel IIa bzw. IIb 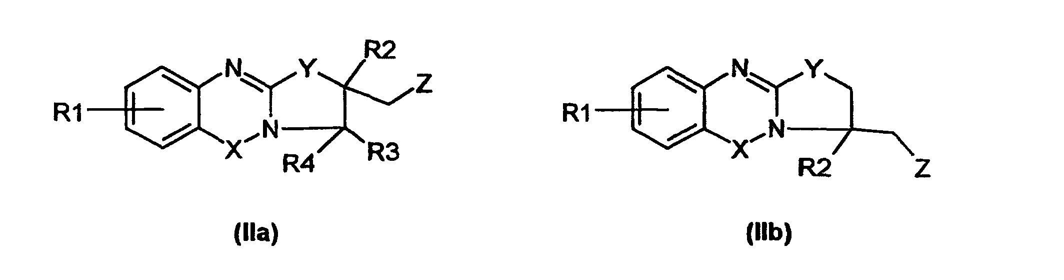 worin bedeuten:
R1 = Wasserstoff, Methyl, Methoxy, Dimethoxy, Hal (Hal = Fluor, Chlor, Brom), Carboxy
R2, R3, R4 = Wasserstoff. Methyl
X = Carbonyl, Sulfonyl
Y = S, O, NH
Z = Chlor , Brom, Jod, Tosyl
sowie deren Tautomere und Salze
mit einem schwefelübertragenden Agens, vorzugsweise Thioharnstoff, in einem inerten Lösungsmittel und anschließender schonender Verseifung der isolierten Zwischenstufen, vorzugsweise der Isothiuroniumsalze, erhalten.

Die Zwischenstufen der allgemeinen Formel IIa und IIb sind literaturbekannt bzw. nach literaturbekannten Syntheseprinzipien erhältlich ( *Chern, J.-W. et al.: Studies on Quinazolines and 1,2,4-Benzothiadiazine 1,1-Dioxides. 8. Synthesis and pharmacological evaluation of tricyclic fused Quinazolines and 1,2,4-Benzothiadiazine 1,1-dioxides as potential α1-Adrenoceptor Antagonists, J. Med. Chem. 41 (1998) 3128-3141; Shiau, C.-Y. et a ,Studies on Quinazolines. 2. Synthesis of 2-(4-Benzylpiperazin-1-ylmethyl)-2,3-dihydro-5H-oxazolo[2,3-b]quinazolin-5-one and -2,3-dihydro-5H-thiazolo[2,3-b]quinazolin-5-one, J. Heterocyclic Chem. 27 (1990) 1467-1472; Kampe, K-D.: Eine einfache Synthese von 5-Oxo-2,3-dihydro-5H-[1,3]oxazolo[2,3-b]chinazolinen, Synthesis 1976, 469- 47).*

### Beispiel 1

### Herstellung von

### (R,S)-2-Mercaptomethyl-2,3-dihydro-5H-thiazolo[2,3-b] chinazolin-5-on (Formel Ia, , R1= R2 = R3 = R4 = Wasserstoff, X= Carbonyl, Y= Schwefel)

10 mmol der literaturbekannten Verbindung (R,S)-2-Brommethyl-3H,5H-thiazolo[2,3-b]chinazolin-5-on, allgemeine Formel IIa, R1= R2 = R3 = R4 = Wasserstoff, X= Carbonyl, Y= Schwefel, Z= Brom, werden in 30 ml Monomethylglycol eingetragen und mit 12 mmol Thiohamstoff unter DC- bzw. HPLC-Kontrolle bei einer Badtemperatur von ca. 100 °C bis zum vollständigen Umsatz erhitzt. Wenn beim Abkühlen keine Kristallisation einsetzt, wird mit Essigester bis zur Trübung versetzt und das sich dann abscheidende Isothiuroniumsalz abgetrennt. Dieses wird in 1 N NaOH unter Zusatz von etwas Ethanol und unter Stickstoffatmosphäre bei einer Badtemperatur von 50 °C verseift. Der Reaktionsverlauf wird DC- bzw. HPLC-chromatographisch verfolgt. Nach beendeter Reaktion wird mit 1 N HCl unter Eiskühlung angesäuert, der sich bildende Niederschlag abgesaugt, mit Wasser gewaschen, getrocknet und bei Bedarf aus Ethanol oder Essigester/ Heptan umkristallisiert.

### Farblose Kristalle

F.: 104-108°C
Ausbeute: 74%
C₁₁H₁₀N₂OS₂ (250)
IR (υ in cm⁻¹): 1605 (C=N), 1662(C=O)
MS m/e (%B): M⁺ 250 (51), 203 (100), 162 (20)

### Beispiel 2

### (R,S)-2-Mercaptomethyl-2-methyl-2,3-dihydro-5H-thiazolo[2,3-b] chinazolin-5-on

### (Formel Ia, , R1= R3 = R4 = Wasserstoff, R2= Methyl, X= Carbonyl, Y= Schwefel)

10 mmol des analog dem literaturbekannten Edukt von Beispiel 1 erhaltenen (R,S)-2-Brommethyl-2-methyl-3H,5H-thiazolo[2,3-b]chinazolin-5-on, allgemeine Formel IIa, R1= R3= R4 = Wasserstoff, R2= Methyl, X= Carbonyl, Y= Schwefel, Z= Brom (farblose Kristalle, F: 116-118°C (Methanol)) werden analog der Vorschrift' in Beispiel 1 umgesetzt.

### Farblose Kristalle.

F.: 110-114°C (Ethanol)
Ausbeute: 54%
C₁₂H₁₂N₂OS₂ (264,4)
IR (υ in cm⁻¹): 1608 (C=N), 1672 (C=O), 2568 (SH)
MS m/e (%B): M⁺ 264(32), 217 (100), 162 (22)

### Beispiel 3

### (R,S)-8-Carboxy-2-mercaptomethyl--2,3-dihydro-5H-thiazolo[2,3-b] chinazolin-5-on

### (Formel Ia, , R1= 8-Carboxy, R2= R3= R4 = Wasserstoff, X= Carbonyl, Y= Schwefel)

10 mmol des analog dem literaturbekannten Edukt von Beispiel 1 erhaltenen (R,S)-2-Brommethyl-8-methoxycarbonyl-3H,5H-thiazolo[2,3-b]chinazolin-5-on, allgemeine Formel IIa, R1=8-Methoxycarbonyl, R2= R3= R4= Wasserstoff, , X= Carbonyl, Y= Schwefel, Z= Brom (ockerfarbener Feststoff, F: 204-208.°C), werden analog der Vorschrift in Beispiel 1 umgesetzt.

### Hellgelber Feststoff.

F.: 214-218 °C (Ethanol)
Ausbeute: 42%
C₁₂H₁₀N₂O₃S₂ (294)
IR (υ in cm⁻¹): 1552 (C=N), 1694 (C=O), 2551 (SH)
MS m/e (%B): M⁺ 294(56), 247 (100), 203 (28)

### Beispiel 4

### (R,S)-3-Mercaptomethyl--2,3-dihydro-5H-thiazolo[2,3-b] chinazolin-5-on

### (Formel Ib, , R1= R2= Wasserstoff, X= Carbonyl, Y= Schwefel)

10 mmol des literaturbekannten (R,S)-3-Brommethyl-3H,5H-thiazolo[2,3-b]chinazolin-5-on, allgemeine Formel IIb, R1= R2= R3Wasserstoff, , X= Carbonyl, Y= Schwefel, Z= Brom werden analog der Vorschrift in Beispiel 1 umgesetzt.

### Farblose Kristalle

F.: 95-98 °C (Ethanol)
Ausbeute: 33%
C₁₁H₁₀N₂OS₂ (250)
IR (υ in cm⁻¹): 1584 (C=N), 1661 (C=O), 2449 (SH)
MS m/e (%B): M⁺ 250(50), 217 (20), 203 (100), 178 (100)/*

### Beispiel 5

### (R,S)-8-Chlor-2-mercaptomethyl-2,3-dihydro-5H-thiazolo[2,3-b] chinazolin-5-on

### (Formel Ia, , R1= 8-Chlor, R2= R3= R4 = Wasserstoff, X= Carbonyl, Y= Schwefel)

10 mmol des analog dem literaturbekannten Edukt von Beispiel 1 erhaltenen (R,S)-2-Brommethyl-8-chlor-3H,5H-thiazolo[2,3-b]chinazolin-5-on, allgemeine Formel IIa, R1=8-Chlor, R2= R3= R4= Wasserstoff, , X= Carbonyl, Y= Schwefel, Z= Brom (hellgelber Feststoff, F: 204-208.°C), werden analog der Vorschrift in Beispiel 1 umgesetzt.

### Farbloser Feststoff.

F.: 134-138°C (Ethanol)
Ausbeute: 56%
C₁₁H₉N₂OS₂C I(284)
IR (υ in cm⁻¹): 1574 (C=N), 1673 (C=O)
MS m/e (%B): M⁺ 284(60), 251 (21), 237 (100), 202 (29)

Die MMP-inhibierende Wirkung der Substanzen wird wie folgt bestimmt:

### Hemmung von Matrix-Metalloproteinase-2 (MMP-2)

MMP-2 (Gelatinase) wird von kultivierten dermalen Fibroblasten in beträchtlichen Mengen in das Kulturmedium abgegeben und ist daher leicht zugänglich. Die sezernierte und inaktive Proform des Enzyms kann durch Trypsinaktivierung oder durch Behandlung mit organischen Quecksilberverbindungen in die enzymatisch aktive Form überführt werden.

Hierzu werden humane dermale Fibroblasten nach etablierten Standardmethoden gewonnen und kultiviert und der zellfreie Kulturüberstand mit Trypsin behandelt. Trypsin wird danach mit einem spezifischen Inhibitor (TLCK) inaktiviert und die aktive MMP-2 durch Affinitätschromatographie an Gelatine-Sepharose und anschließender Gelfiltration an Sepharose teilgereinigt. Die Identifizierung und Charakterisierung von MMP-2 erfolgte durch die Verfügbarkeit eines kommerziellen Immuntests.

### Präparation der humanen Kollagenase MMP-9

Native MMP-9 wurde reproduzierbar und in guter Ausbeute und Reinheit aus humanem Buffy Coat gewonnen. Hierzu wird Buffy Coat mit 10% (v/v) Triton X-100 auf eine Endkonzentration von 0,4% gebracht, 30 min. auf Eis geschüttelt und dann 1 Vol. 2-fach Bindungspuffer (40 mM Tris-HCl pH 7,5, 10 mM CaCl₂, 1 M NaCl, 0,2% (v/v) Triton X-100) hinzugefügt und weitere 30 min. auf Eis geschüttelt.

Die Lösung wird 15 min. bei 16 000 Upm an einem SS-34 Rotor bei 4°C abzentrifugiert und über Glaswolle filtriert. Das Filtrat wird mit Gelatine-Agarose, welche mit Bindungspuffer äquilibriert wurde, gebatcht und 1 Std. auf Eis geschüttelt.

Die beladene Gelatine-Agarose wird in eine Säule überführt und mit mindestens 10 Vol. Bindungspuffer proteinfrei gespült. Die Elution der gebundenen MMP-9 erfolgt mit 2 Gelvolumen Bindungspuffer plus 5% (v/v) DMSO.

Das Eluat kann zum Puffer-Austausch und gleichzeitiger Abtrennung geringer Verunreinigungen an MMP-2 über eine Gelfiltration an Sephadex G-75 aufgetrennt werden. Hierzu wird der Puffer I (20 mM Tris-HCl pH 7,5, 5 mM CaCl₂, 100 mM NaCl, 0,1% (v/v) Triton X-100) verwendet.

Das so gewonnene Eluat enthällt MMP-9 in den drei bekannten Zustandsformen: Monomer, Homodimer, Heterodimer. Die Reinheit des Enzyms beträgt ca. 90%, wobei die restlichen Fremdproteine Fibronektin und äußerst geringe Mengen an TIMP's sind.

Das latente Enzym wird durch 30 - 60 min. Inkubation bei 37°C mit 1/100 Vol. Trypsin (10 mg/ml) aktiviert. Trypsin wird durch Zugabe von 1 mM PMSF oder mit einem spezifischen Inhibitor (TLCK) gehemmt.

### Klonierung und Expression der katalytischen Domäne der MMP-8

Die Nutzung der katalytischen Domäne der MMP-8 als weiteres Testenzym wurde deshalb gewählt, da es eine hohe Stabilität besitzt und darüber hinaus als aktives Enzym vorliegt und deshalb nicht aktiviert werden braucht, was als eine der häufigsten Fehlerursachen in Frage kommt, da die zur Aktivierung genutzten Quecksilberverbindungen häufig mit dem Testsystem bzw. dem Enzym interferieren und dadurch die Messergebnisse verfälschen können. Die Klonierungsstrategie richteten wir danach aus, dass wir statt des Gesamtenzyms nur dessen enzymatisch aktive katalytische Domäne in E. coli klonierten. Mit der konstruierten katalytischen Domäne der MMP-8, erzielten wir ein stabiles, enzymatisch aktives und hochreines Enzym, welches für die Routineuntersuchungen der inhibitorischen Aktivität der synthetisierten Inhibitoren sehr gut geeignet war und die Messergebnisse der einzelnen Messserien absolut vergleichbar waren.

Die Klonierung und Expression der rekombinanten katalytischen Domäne der MMP-8 wurde entsprechend den Angaben von SCHNIERER et al. (Schnierer S, Kleine T, Gote T, Hillemann A Knäuper V, Tschesche H: The recombinant catalytic domain of human neutrophil collagenase lacks type I collagen substrate specificity. Biochem Biophys Res Comm (1993) vol. 191 No. 2, 319-326) durchgeführt.

### Quantitativer Fluoreszenz-Assay für Matrix-Metalloproteinasen

Durch enzymatische Spaltung des synthetischen Substrates Mca-Pro-Leu-Gly-Leu-Dpa-Ala-Arg-NH₂ durch die jeweilige Kollagenase, wird die fluoreszente Gruppe Mca vom internen Quencher Dpa getrennt. Dabei erfolgt eine starke Zunahme der Fluoreszenz im Messansatz, die am Fluorimeter quantifiziert werden kann (λₑₓ 328 nm, λₑₘ 393 nm) und innerhalb der ersten Minuten linear verläuft. Eine gewisse Spezifität des Tests für Matrix-Metalloproteinasen ergibt sich einerseits aus der Aminosäuresequenz -Pro-Leu-Gly-Leu- im Substrat und andererseits durch die gewählten Inkubationsbedingungen. Matrix-Metalloproteinasen spalten das Substrat an der Bindung Gly-Leu. Gemessen wird die proteolytische Restaktivität vorinkubierter Ansätze von Enzym und Inhibitor, wobei die Substrat- und Enzymkonzentration konstant gehalten und die Inhibitorkonzentration variiert wurde. Es wurden für jeden getesteten Inhibitor drei Messreihen mit unterschiedlicher Substratkonzentration aufgenommen. Aus der zeitabhängigen Fluoreszenzzunahme wird die Enzymaktivität in Fluoreszenzeinheiten pro min berechnet. Die Bestimmung der Kᵢ-Werte erfolgte graphisch nach der Methode von DIXON (1953) durch Auftragung der reziproken Reaktionsgeschwindigkeit 1/v (y-Achse) gegen Inhibitorkonzentration (x-Achse).

### Assay

1984 µl Messpuffer (100mM Tris-HCl pH 7,5, 100 mM NaCl, 10 mM CaCl₂, 0,05% Brij 35)
2 µl Inhibitor, gelöst in DMSO, bzw. DMSO allein (nichtinhibierender Ansatz)
4 µl Enzym (MMP-2 oder MMP-9 oder katalytische Domäne der MMP-8)
• 5 min. Vorinkubation des Enzym-Inhibitor-Gemischs bei Raumtemperatur unter Rühren
• Start der Reaktion mit 10 µl Substrat gelöst in DMSO
• Aufzeichnung der zeitabhängigen Fluoreszenzzunahme über 2 min.

| **Hemmung von humanen MMPs durch die erfindungsgemäßen Inhibitoren der allgemeinen Formeln Ia und Ib** | | | | |
|---|---|---|---|---|
| **Verbindung nach Beispiel** | **MMP-2** (Kᵢ-Wert) | **MMP-8** (Kᵢ-Wert) | **MMP-9** (Kᵢ-Wert) | **MT1-MMP** (Kᵢ-Wert) |
| 1 | 70% Hemmung bei 10 µM* | 80% Hemmung bei 10 µM * | 0.5 µM | 50% Hemmung bei 2.5 µM* |
| 2 | 50% Hemmung bei 10 µM* | 85% Hemmung bei 10 µM * | 1.0 µM | 4.0 µM |
| 3 | 25% Hemmung bei 10 µM* | 24 µM | 15 µM | 21 µM |
| 4 | 50% Hemmung bei 10 µM* | 25% Hemmung bei 10 µM * | 75% Hemmung bei 10 µM * | 55 µM |
| 5 | 80% Hemmung bei 10 µM* | 90% Hemmung bei 10µM* | 85% Hemmung bei 10 µM* | 85% Hemmung bei 10 µM* |

| | | | | |
|---|---|---|---|---|
| *) Bestimmung eines expliciten Kᵢ-Wertes nicht möglich, da nichtlinearer Dixon-Plot | | | | |

## Patentansprüche

1. Thiazolo[2,3-b]chinazolinone, Oxazolo[2,3-b]chinazolinone, Imidazo[2,1-b] chinazolinone der allgemeinen Formeln Ia und Ib 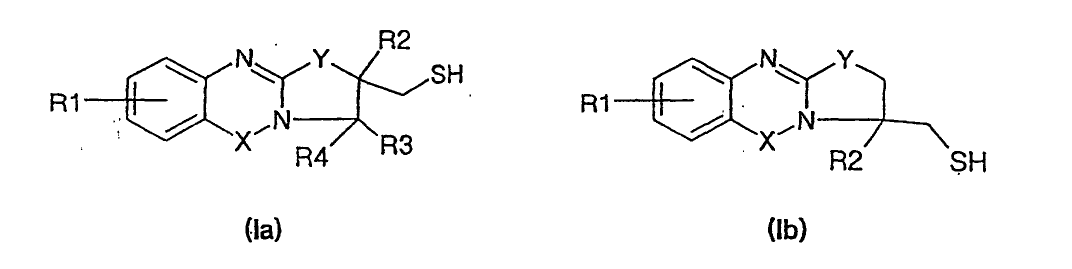 worin bedeuten
R1 = H, Methyl, Methoxy, Dimethoxy, Hal (Hal = F, Cl, Br), Carboxy
R2, R3, R4 = Wasserstoff, Methyl
X = Carbonyl, Sulfonyl,
Y = S, O, NH
sowie deren Tautomere und Salze.

2. Verfahren zur Herstellung der Verbindungen nach Anspruch 1,
**dadurch gekennzeichnet, dass**
Verbindungen der allgemeinen Formeln IIa und IIb 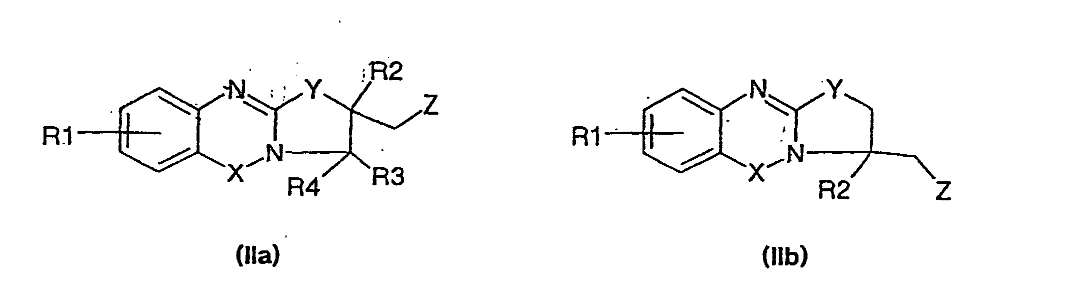 worin bedeuten
R1 = H, Methyl, Methoxy, Dimethoxy, Hal (Hal = F, Cl, Br), Carboxy
R2, R3, R4 = Wasserstoff, Methyl
X = Carbonyl, Sulfonyl
Y= S, O, NH
Z = Cl, Br, J, Tosyl
mit einem schwefelübertragenden Agens in einem inerten Lösungsmittel umgesetzt werden,
das entstandene Produkt isoliert wird
und durch anschließende schonende Verseifung
das gewünschte Endprodukt erhalten wird.

3. Verfahren nach Anspruch 2, **dadurch gekennzeichnet, dass** als schwefelübertragendes Agens Thioharnstoff oder Xanthogenate eingesetzt wird.

4. Verwendung von Verbindungen nach Anspruch 1 als Matrix-Metalloproteinase-Inhibitoren.

5. Pharmazeutische Zusammensetzung, enthaltend Verbindungen gemäß Anspruch 1.

## Claims

1. Thiazolo[2,3-b]quinazolinones, oxazolo[2,3-b]quinazolinones, imidazo[2,1-b]quinazolinones of general formulae Ia and Ib 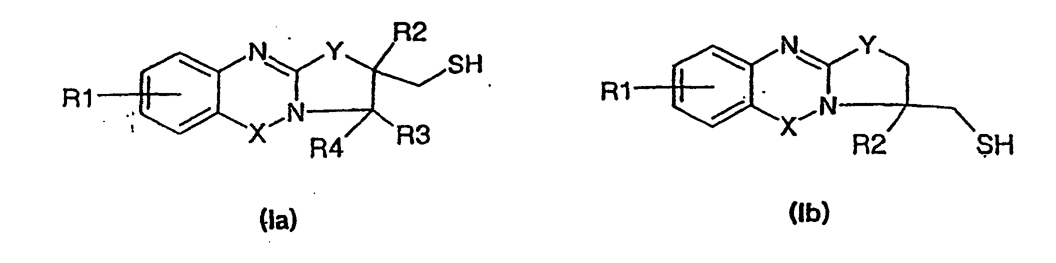 wherein
R1 = H, methyl, methoxy, dimethoxy, Hal (Hal = F, Cl, Br), carboxy,
R2, R3, R4 = hydrogen, methyl
X = carbonyl, sulfonyl,
Y = S, 0, NH
and the tautomers and salts thereof.

2. A method for the production of the compounds according to claim 1,
**characterized in that**
compounds of general formulae IIa and IIb 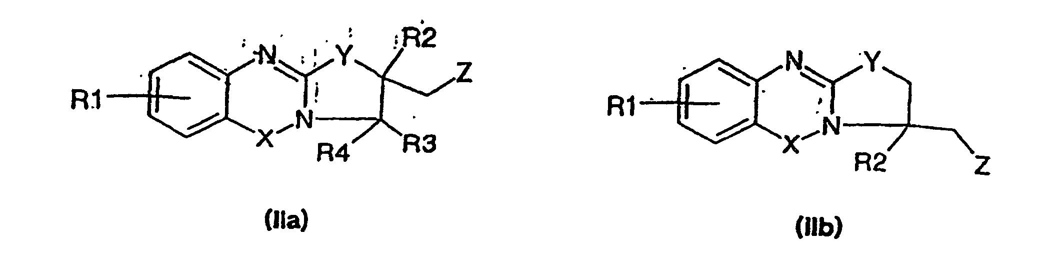 wherein
R1 = H, methyl, methoxy, dimethoxy, Hal (Hal = F, Cl, Br), carboxy
R2, R3, R4 = hydrogen, methyl
X = carbonyl, sulfonyl
Y = S, 0, NH
Z = Cl, Br, J, tosyl
are reacted with a sulfur-transferring agent in an inert solvent,
the resulting product is isolated,
and the desired end product is obtained
by subsequent mild saponification.

3. The method according to claim 2, **characterized in that** thiourea or xanthogenates are used as the sulfur-transferring agent.

4. Use of compounds according to claim 1 as matrix-metalloproteinase inhibitors.

5. Pharmaceutical composition containing compounds according to claim 1.

## Revendications

1. Thiazolo [2,3-b] chinazolinone, oxazolo [2,3-b] chinazolinone, imidazo [2,1-b] chinazolinone de formules générales la et Ib 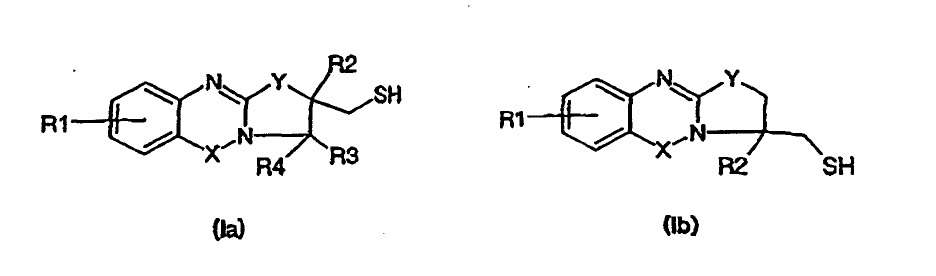 dans lesquelles
R1 = H, méthyle, méthoxy, diméthoxy, Hal (Hal = F, Cl, Br), carboxy
R2, R3, R4 = hydrogène, méthyle
X = carbonyle, sulfonyle
Y = S, O, NH
ainsi que leurs tautomères et sels.

2. Procédé pour la préparation de composés selon la revendication 1, **caractérisé en ce que** les composés de la formule générale IIa et IIb 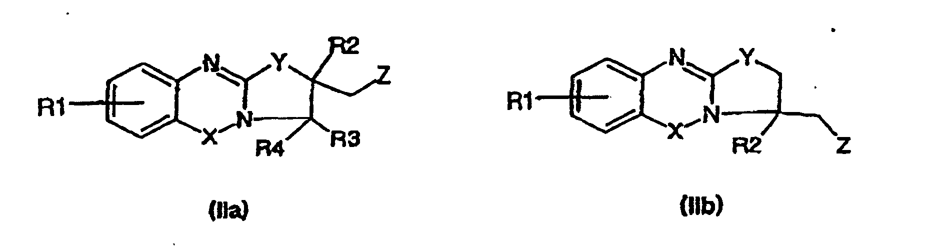 dans lesquelles
R1 = H, méthyle, méthoxy, diméthoxy, Hal (Hal = F, Cl, Br), carboxy
R2, R3, R4 = hydrogène, méthyle
X = carbonyle, sulfonyle
Y = S, O, NH
Z = Cl, Br, J, Tosyl
réagissent dans un solvant inerte avec un agent donneur de soufre
le produit formé est isolé
et le produit final désiré est obtenu par saponification douce appropriée.

3. Procédé selon revendication 2, **caractérisé en ce qu'**on utilise la thiourée ou le xanthogénate comme agent donneur de soufre

4. Utilisation de composés selon la revendication 1 comme inhibiteurs de la métalloprotéinase de matrice.

5. Compositions pharmaceutiques contenant les composés selon la revendication 1.
